# EUROPÄISCHE PATENTSCHRIFT

(11) **EP 1 604 966 B1**
(45) Veröffentlichungstag und Bekanntmachung des Hinweises auf die Patenterteilung: **16.01.2013**
(21) Anmeldenummer: 05011673.0
(22) Anmeldetag: 31.05.2005
(51) Int. Cl.: C07C 29/141, C07C 29/16, C07C 31/27, C07C 47/347

(54) **Verfahren zur Herstellung von Tricyclo-[5.2.1.0 2,6]-decandimethylol**
Process for the preparation of tricyclo-[5.2.1.0 2,6]-decane dimethylol
Procédé de préparation tricyclo-[5.2.1.0 2,6]-décène diméthylol

(30) Priorität: 08.06.2004 DE 102004027955
(43) Veröffentlichungstag der Anmeldung: 14.12.2005
(73) Patentinhaber: OXEA GmbH, 46147 Oberhausen (DE)
(72) Erfinder: Dukat, Wolfgang, Dr., 46145 Oberhausen (DE); Storm, Edgar, 46147 Oberhausen (DE); Schmid, Klaus, Dr., 46539 Dinslaken (DE)

(56) Entgegenhaltungen:
- EP-A- 1 065 194
- DE-B- 1 030 322
- DE-B- 1 034 167
- GB-A- 1 170 226
- CORNILS B ET AL: "DERIVATE DES DICYCLOPENTADIENS - AKTUELLE SCHLUESSELVERBINDUNGEN" CHEMIKER ZEITUNG, HUETHIG VERLAG, HEIDELBERG, DE, Bd. 98, Nr. 2, 1974, Seiten 70-76, XP009022594 ISSN: 0009-2894

## Beschreibung

Die vorliegende Erfindung betrifft ein Verfahren zur Herstellung von TCD-Alkohol DM {3(4), 8(9)-Dihydroxymethyl-tricyclo[5.2.1.0^{2.6}]decan} aus Dicyclopentadien (DCP).

Das durch Dimerisierung von Cyclopentadien leicht zugängliche und auch großtechnisch hergestellte Dicyclopentadien (DCP) lässt sich zu anwendungstechnisch wichtigen Verbindungen umsetzen, denen das Tricyclodecan-Gerüst besondere Eigenschaften verleiht. Die vom DCP-abgeleiteten Verbindungen mit Tricyclodecan-Struktur werden in der Literatur häufig unterschiedlich bezeichnet. In Anlehnung an die aus Chemiker-Zeitung, 98, 1974, Seiten 70 bis 76 bekannte Nomenklatur für DCP-Derivate, wird auch im folgenden die auf dem Tricyclodecan-Gerüst, auch TCD-Gerüst genannt, aufbauende Nomenklatur verwendet.

TCD-Alkohol DM {3(4), 8(9)-Dihydroxymethyl-tricyclo[5.2.1.0^{2.6}]decan} besitzt als wichtiges Zwischenprodukt für die chemische Industrie eine große wirtschaftliche Bedeutung. Der zweiwertige Alkohol ist in vielfältiger Weise für unterschiedliche Anwendungen von hohem technischen Interesse: Acrylsäureester bzw. Methacrylsäureester von OH-gruppenhaltigen tricyclischen Decanolen (DE 22 00 021), als Bestandteil von bei Sauerstoffausschluss erhärtenden Acrylsäureesterklebstoffen, (Meth)acrylsäureester von Ethergruppen enthaltenden tricyclischen Decanolen (EP 23 686), zur Herstellung von Kleb- und Dichtungsstoffen, Ester und Polyester der Tricyclodecanreihe (DE 934 889), die als Weichmacher und hochwertige Esterschmieröle geeignet sind, Riechstoffkompositionen (DE-OS 2 307 627) und säuresterilisationsfeste Polyesterlacke (DE 31 34 640) im Metall-Lackierungsbereich. TCD-Alkohol DM erhält man durch Hydrierung der Hydroformylierungsprodukte des Dicyclopentadiens, der sogenannten TCD-Aldehyde.

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Kobalt als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Wegen der Gefahr der Retro-Diels-Alder-Reaktion bei den Temperaturen der Oxo-Synthese und der damit verbundenen Freisetzung von Cyclopentadien, das zur Komplexbildung gegenüber Übergangsmetallen befähigt ist und das die Aktivität der eingesetzten Katalysatoren mindern kann, muss die Hydroformylierung unter besonderen Bedingungen ablaufen. Es erwies sich als vorteilhaft, den früher üblichen Kobalt-Katalysator durch Rhodium zu ersetzen, das eine hohe Selektivität der Umsetzung zu Aldehyden erreichen lässt und die Hydroformylierung bei Bedingungen erlaubt, bei denen das Ausmaß der Retro-Diels-Alder-Spaltung geringer ist. Eine zusammenfassende Darstellung über die Hydroformylierung von Dicyclopentadien und über die Weiterverarbeitung der TCD-Aldehyde findet sich in Chemiker-Zeitung 98, 1974, 70-76. Besondere Bedeutung besitzt dabei 8(9)-Formyl-tricyclo[5.2.1.0^{2.6}]dec-3-en, auch als TCD-Monenal bezeichnet und 3(4),8(9)-Bisformyl-tricyclo[5.2.1.0^{2.6}]decan, auch als TCD-Dialdehyd bezeichnet. Wegen ihrer thermischen Labilität, die bei der Destillation zu Verlusten führt, werden die TCD-Aldehyde zumeist nicht rein isoliert, sondern als Rohprodukte der Hydroformylierungsreaktion weiterverarbeitet.

So ist beispielsweise aus GB 1,170,226 bekannt, das unter Rhodium-Katalyse erhaltene Hydroformylierungsgemisch der Dicyclopentadien-Hydroformylierung entweder nach Abtrennung des rhodiumhaltigen Katalysators in Gegenwart eines gängigen Hydrierkatalysators zu hydrieren oder aber das Reaktionsgemisch ohne Abtrennung des Rhodiumkatalysators und ohne Zusatz eines weiteren Hydrierkatalysators bei erhöhter Temperatur in Gegenwart von Synthesegas in TCD-Alkohol-DM zu überführen.

Nach EP-A2-0 348 832, das die Herstellung von 3(4),8(9)-Bis(aminomethyl)-tricyclo [5.2.1.0^{2.6}]-decan (TCD-Diamin) betrifft, wird das rhodiumhaltige Reaktionsgemisch der Dicyclopentadien-Hydroformylierung ohne Abtrennung des Katalysators der nachfolgenden reduktiven Aminierung zugeführt, wobei sich das in der Hydroformylierungsstufe eingesetzte Rhodium nahezu vollständig auf dem Hydrierkatalysator abscheidet.

Aufgrund der vielseitigen Anwendungsmöglichkeiten besitzt TCD-Alkohol-DM ein hohes wirtschaftliches Interesse und in der Patentliteratur finden sich auch zahlreiche Hinweise auf Verfahren zu seiner Herstellung.

Das US-Patent US 4 647 708 beschreibt die Hydroformylierung von Dicyclopentadien mit Rh als Katalysator in Gegenwart von Ionenaustauschern (Dowex® MWA-1) in Toluol / THF als Lösungsmitteln. Die Umsetzung erfolgt bei 120°C und 27,5 MPa CO/H₂ (im Verhältnis 1 : 2) in zwei getrennten kontinuierlich betriebenen Autoklaven. Anhand der offenbarten Versuchsergebnisse kann man ersehen, dass die Ausbeute an TCD-Alkohol DM in dem 30 tägigen Versuchslauf von 85 % auf 65 % abfällt. Das Reaktionssystem ist somit für eine technische Anwendung nicht geeignet.

Im US-Patent US 4 262 147 wird der Einsatz von bimetallischen Rh/Co-Clustern auf Harzen wie Amberlite® IRA-68 beschrieben. Unter den angewandten Bedingungen (110°C, 11 MPa, 8 Stunden) wird eine Selektivität von 68 % an TCD-Alkohol DM in dieser einstufigen Synthese erhalten.

Ein modifiziertes Co-Verfahren wird in der DE-PS 3 008 497 beschrieben, wobei Dicyclopentadien unter Katalyse von Co/Tri-n-octylphosphin bei 200°C und einem Synthesegasdruck von 15 MPa umgesetzt wird. Nach einer Reaktionszeit von 5 Stunden wird der TCD-Alkohol DM in einer Ausbeute von 69 % erhalten. Als Nebenprodukte entstehen 11,7 % des TCD-Monoalkohols und 14,6 % Hydroxymethylcyclopentan. Aufgrund der notwendigerweise angewandten hohen Temperaturen kommt es zur Retro-Diels-Alder-Reaktion von Dicyclopentadien in Cyclopentadien und somit zu der Bildung signifikanter Mengen an Hydroxymethylcyclopentan. Daher ist diese Verfahrensvariante für eine technische Anwendung nicht geeignet.

Aus DE 10 30 322 B ist ein unter Kobaltkatalyse durchgeführtes Hydroformylierungsverfahren von Dicyclopentadien bekannt. Der erhaltene Dialdehyd wird anschließend in Gegenwart von Wasser katalytisch hydriert. Auch DE 1 034 167 B behandelt die Hydroformylierung von Dicyclopentadien in Gegenwart von Kobaltkatalysatoren. Der erhaltene Aldehyd wird zunächst bei erhöhter Temperatur einer Druckwasserbehandlung unterworfen und anschließend hydriert.

Aus JP 111 00 339 ist bekannt, die Hydroformylierung von DCP in Isopropanol/Toluol mit Rhodiumdicarbonylacetylacetonat, Tris(2,4-di-tert.-butylphenyl)-phosphit und Triethylamin bei 120°C unter 8,8 MPa Synthesegas über 8 Stunden durchzuführen. Es werden 93 % TCD-Dialdehyd erhalten, der in Isopropanol bei 110°C und 0,78 MPa Wasserstoff für 6 Stunden an Raney-Nickel zu 91 % TCD-Alkohol DM hydriert wird. Der Einsatz der komplexen und aufwendig herstellbaren Phosphit-Liganden ist für eine technische Anwendung und auch aus wirtschaftlichen Gründen von Nachteil. Zudem wird die breite Verwendung von Phosphit-Liganden durch ihre im Vergleich zu konventionellen Phosphin-Liganden geringere Stabilität und höhere Hydrogegenüber Wasser und -Säurespuren eingeschränkt und die während des kontinuierlich betriebenen Hydroformylierungsprozesses gebildeten Phosphonigsäuren beeinträchtigen die Katalysatorlebensdauer und müssen aufwendig aus dem Prozess entfernt werden. Außerdem ist bei der Verwendung von Aminen stets mit einer Verunreinigung des TCD-Alkohol DM mit stickstoffhaltigen Komponenten zu rechnen.

In der EP 1 065 194 wird ein Niederdruckverfahren zur Hydroformylierung von Dicyclopentadien beschrieben, in dem als Katalysatorsystem ebenfalls Rh/Tris-(2,4-di-tert.-butylphenyl)-phosphit eingesetzt wird. Die Hydroformylierung wird bei Drücken von 1 - 15 MPa und Temperaturen von 80 - 140°C durchgeführt. Als Lösungsmittel werden inerte Kohlenwasserstoffe wie Toluol, Diisopropylbenzol oder Methylcyclohexan verwendet. Die Aufarbeitung des Hydroformylierungsprodukts erfolgt durch eine mehrstufige Extraktion unter Verwendung von mehrwertigen Alkoholen wie z.B. Ethylenglykol, wobei der Zusatz von tertiären Aminen empfohlen wird. Nach der Extraktion liegt das Oxorohprodukt vorwiegend in der Alkoholphase vor, während sich geringe Anteile an Mono- und Dialdehyd sowie die Hauptmenge an Rhodium und Phosphin-Liganden in der Kohlenwasserstoffphase befinden. Es wird darauf hingewiesen, dass die Extraktion in absoluter Abwesenheit von Sauerstoff erfolgen muss. Der Einsatz von Extraktionsmitteln unter Zusatz von tertiären Aminen sowie die absolute Notwendigkeit der Abwesenheit von Sauerstoff erschweren die technische Durchführung dieses Verfahrens und beinhalten das Risiko der Verunreinigung von TCD-Alkohol DM mit Spuren von Aminen.

Die bekannten Verfahren zur Herstellung von TCD-Alkohol DM durch Hydroformylierung von Dicyclopentadien mit nachfolgender Hydrierung erfordern entweder die Anwesenheit von speziellen, in der Technik nicht verfügbaren oder umweltunverträglichen Katalysatorsystemen oder ermöglichen nur wirtschaftlich unbefriedigende Selektivitäten und Ausbeuten an TCD-Alkohol Alkohol DM. Daher besteht ein Bedarf nach einem möglichst einfachen und kostengünstigen Verfahren zur Herstellung von TCD-Alkohol DM.

Die Erfindung besteht daher in einem Verfahren zur Herstellung von 3(4),8(9)-Dihydroxymethyl-tricyclo[5.2.1.0^{2.6}]decan durch Hydrierung der Hydroformylierungsprodukte des Dicyclopentadiens. Es ist dadurch gekennzeichnet, dass man die Hydroformylierungsprodukte in homogener organischer Phase in Gegenwart von homogen gelösten Rhodiumkatalysatoren herstellt und ohne weitere Reinigung, ohne Katalysatorabtrennung und ohne Lösungsmittelabtrennung die Hydrierung in Gegenwart von Nickelkatalysatoren nach Zusatz von Wasser durchführt.

Überraschenderweise wurde gefunden, dass die Gegenwart von Wasser in der Hydrierstufe zu einer deutlichen Ausbeuteverbesserung an TCD-Alkohol DM führt. Dabei kann Wasser direkt in dem in die Hydrierstufe eingesetzten rohen Hydroformylierungsprodukt der Dicyclopentadienhydroformylierung zugegen sein oder aber Wasser kann dem rohen Hydroformylierungsprodukt vor oder während der Hydrierstufe zugesetzt werden.

Die Hydroformylierung von Dicyclopentadien führt man in homogener organischer Phase in Gegenwart von Rhodiumkatalysatoren durch, die in dem organischen Reaktionsgemisch löslich sind. Die Hydroformylierungsreaktion kann entweder nach konventionellen Verfahren in Abwesenheit von Wasser durchgeführt werden. Man kann aber auch dem homogenen Hydroformylierungsgemisch Wasser zusetzen. Setzt man der organischen Phase Wasser zu, so wird die eingesetzte Wassermenge im allgemeinen durch die Wasserlöslichkeit im dem organischen Reaktionsgemisch bestimmt. Es ist jedoch nicht ausgeschlossen, Wasser dem Reaktionsgemisch in einer Menge über die Löslichkeitsgrenze hinaus zuzusetzen.

Der Wasserzusatz liegt im Allgemeinen bei wenigstens 0,1 Gew.-%, vorzugsweise bei wenigstens 0,5 Gew.-%, jeweils bezogen auf den Gesamteinsatz.

Wird Wasser über die Löslichkeitsgrenze hinaus dem Reaktionsgemisch zugesetzt, ist es vor der Durchführung der Hydroformylierungsstufe vorteilhaft, abgeschiedenes Wasser zu entfernen, vorzugsweise über eine Phasentrennung.

Die Hydroformylierungsstufe führt man in einem homogenen Reaktionssystem durch. Der Begriff homogenes Reaktionssystem steht für eine im Wesentlichen aus Lösungsmittel, Katalysator, Ausgangsstoffe, Reaktionsprodukt und zugesetztem Wasser zusammengesetzte homogene Lösung. Es wird mit einem Lösungsmittelzusatz gearbeitet. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffnöl, cyclische, geradkettige oder verzweigte Kohlenwasserstoffe wie Cyclohexan, n-Hexan, n-Heptan oder n-Octan, Ether, wie Tetrahydrofuran, Ketone oder Texanol® der Firma Eastman. Dem Lösungsmittel können weitere Verdünnungsmittel, wie aliphatische Alkohole, beispielsweise 2-Ethylhexanol oder iso-Butanol zugesetzt werden. Der Anteil des Lösungsmittels im Reaktionsmedium kann über einen weiten Bereich variiert werden und beträgt üblicherweise zwischen 10 und 90 Gew.-%, vorzugsweise 20 bis 50 Gew.-%, bezogen auf das Reaktionsgemisch.

Falls Wasser dem organischen Hydroformylierungsgemisch zugesetzt wird, ist die Wassermenge vorteilhafterweise so zu bemessen, dass nach Wasserzusatz noch eine homogene flüssige organische Phase vorliegt und sich keine separate Wasserphase abscheidet. Die Löslichkeitsgrenze von Wasser in dem organischen Reaktionsgemisch hängt von verschiedenen Einflußfaktoren ab, beispielsweise von der Polarität des verwendeten Lösungsmittels und seinem Anteil im Reaktionsgemisch und kann durch einfache Versuche ermittelt werden. Wasserzusätze von weniger als 0,1 Gew.-%, bezogen auf die gesamte Einsatzmenge, bewirken keinen vorteilhaften Effekt mehr und sind daher entbehrlich und verkomplizieren unnötig die Reaktionsführung.

Verwendet man aliphatische oder aromatische Kohlenwasserstoffe, wie Toluol oder Cyclohexan als Lösungsmittel, so beträgt in einer bevorzugten Ausgestaltung des erfindungsgemäßen Verfahrens der Wassergehalt 0,5 bis 2,5, vorzugsweise 1,0 bis 2,0 Gew.-%, bezogen auf den Gesamteinsatz.

Als Katalysatoren in der Hydroformylierungsstufe verwendet man Rhodium-verbindungen. Dabei werden im allgemeinen Rhodium-Verbindungen eingesetzt, die nicht mit phosphorhaltigen Liganden, wie Phosphinen oder Phosphiten modifiziert sind. Solche, nicht mit Phosphinen oder Phosphiten modifizierten Rhodiumkatalysatoren und ihre Eignung als Katalysator zur Hydroformylierung sind aus der Literatur bekannt und sie werden als unmodifizierte Rhodiumkatalysatoren bezeichnet. Es wird in der Fachliteratur angenommen, dass die Rhodium-Verbindung HRh(CO)₄ die katalytisch aktive Rhodiumspezies bei der Hydroformylierung mit unmodifizierten Rhodiumkatalysatoren ist, obgleich dies aufgrund der vielen in der Hydroformylierungszone nebeneinander ablaufenden Chemismen nicht eindeutig bewiesen ist. Da im allgemeinen die Verwendung von nicht mit Phosphinen modifizierten Rhodiumkatalysatoren einen geringeren Rhodiumgehalt erfordert, arbeitet man vorzugsweise in der Hydroformylierungsstufe mit unmodifizierten Rhodiumkatalysatoren. Der Rhodiumgehalt beträgt im allgemeinen von 5 bis 100 ppm, bezogen auf das homogene Reaktionsgemisch.

Man kann aber auch in der Hydroformylierungsstufe Rhodium-Komplexverbindungen verwenden, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von etwa 5 bis etwa 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphor-Liganden durchzuführen, der mit Rhodium keine Komplexverbindung mehr eingeht. Der freie Phosphor-Ligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylphenylphosphine, Cycloalkylphenylphosphine und organische Diphosphite. Wegen seiner leichten Zugänglichkeit wird Triphenylphosphin besonders häufig angewandt.

Arbeitet man mit einem modifizierten Rhodium-Komplexkatalysatorsystem, beträgt üblicherweise in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Die Hydroformylierung des Dicyclopentadiens mit Kohlenmonoxid und Wasserstoff erfolgt bei Temperaturen von 70 bis 150°C. Bevorzugt hält man Temperaturen von 80 bis 140°C und insbesondere von 100 bis 140°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung und Synthesegas unter den Bedingungen der Hydroformylierungsreaktion im Reaktionsgemisch, gegebenenfalls in Gegenwart von organischen Phosphor(III)-Verbindungen. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators gelangt Rhodium entweder in metallischer Form oder als Verbindung zum Einsatz. In metallischer Form verwendet man Rhodium entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-Ethylhexanoat, xanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆ oder Übergangsmetall-Komplexverbindungen, z.B. Cyclopentadienylrhodiumverbindungen, Rhodiumacetylacetonat oder [RhCl(Cyclooctadien-1,5]₂ eingesetzt werden. Rhodiumhalogenverbindungen kommen wegen ihres korrosiven Verhaltens der Halogenidionen weniger in Betracht.

Als besonders geeignet hat sich Rhodiumoxid, Rhodiumacetat und Rhodium-2-ethylhexanoat erwiesen.

Die Hydroformylierungsstufe kann sowohl absatzweise als auch kontinuierlich durchgeführt werden.

Die Umsetzungsprodukte der Dicyclopentadienhydroformylierung werden ohne weitere Reinigung, ohne Katalysatorabtrennung und ohne Lösungsmittelabtrennung der Hydrierstufe zugeführt. Eine Abtrennung des zuvor in der Hydroformylierungsstufe anwesenden und/oder zugesetzten Wassers unterbleibt.

Ein Wasserzusatz bei der in homogener Phase unter Verwendung von in dem organischen Reaktionsgemisch homogen löslichen Rhodiumkatalysatoren durchgeführten Hydroformylierungsstufe ist aber nicht zwingend erforderlich. Dabei wird zunächst Dicyclopentadien nach konventionellen Verfahren im homogenen Reaktionsmedium hydroformyliert.

Nach dem erfindungsgemäßen Verfahren wird die Hydrierung der Hydroformylierungsprodukte des Dicyclopentadiens nach Zusatz von Wasser durchgeführt. Der Wasserzusatz kann vor oder während der Hydrierung zum TCD- Alkohol DM erfolgen.

Da der eingesetzte rohe TCD-Dialdehyd eine im Vergleich zum Dicyclopentadien höhere Polarität aufweist, erhöht sich die Löslichkeitsgrenze des zugesetzten Wassers in dem Reaktionsgemisch und Wasser kann in einer höheren Menge zugesetzt werden, ohne dass sich eine separate Wasserphase abscheidet. Wasserzusätze unterhalb von 0,1 Gew.-%, bezogen auf die gesamte Einsatzmenge, bewirken keinen technischen Vorteil. Vorzugsweise setzt man Wasser dem Reaktionsgemisch auf einen Gehalt von 0,5 bis 5,0 Gew.-%, insbesondere von 1,0 bis 4,0 Gew.-%, bezogen auf die gesamte Einsatzmenge, zu.

Es ist aber ebenfalls möglich, Wasser bis über die Löslichkeitsgrenze hinaus dem aus der Hydroformylierungsstufe resultierenden Reaktionsgemisch zuzusetzen.

Die Hydrierung des rohen TCD-Dialdehyds zum TCD-Alkohol DM erfolgt unter allgemein üblichen Reaktionsbedingungen in Gegenwart konventioneller Nickelkatalysatoren als Hydrierkatalysatoren. Im Allgemeinen beträgt die Hydriertemperatur 70 bis 170°C und der angewandte Druck 1 bis 30 MPa.

Das katalytisch aktive Metall Nickel kann auf einem Träger aufgebracht werden, im Allgemeinen in einer Menge von etwa 5 bis etwa 70 Gew.-%, vorzugsweise etwa 10 bis etwa 65 Gew.-% und insbesondere etwa 20 bis etwa 60 Gew.-% jeweils bezogen auf das Gesamtgewicht des Katalysators. Als Katalysatorträger eignen sich alle herkömmlichen Trägermaterialien, z.B. Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die z.B. der Verbesserung ihrer Hydrieraktivität und/oder ihrer Standzeit und/oder ihrer Selektivität dienen. Derartige Zusatzstoffe sind bekannt, zu ihnen gehören z.B. die Oxide des Natriums, Kaliums, Magnesiums, Calciums, Bariums, Zinks, Aluminiums, Zirconiums und Chroms. Sie werden dem Katalysator im Allgemeinen in einem Anteil von insgesamt 0,1 bis 50 Gew.-Teile bezogen auf 100 Gew.-Teile Nickel zugesetzt.

Aber auch trägerfreie Katalysatoren, wie Raney-Nickel können in dem Hydrierprozess verwendet werden.

Die Hydrierstufe wird diskontinuierlich oder kontinuierlich in flüssiger Phase mit suspendierten Katalysatoren oder in flüssiger oder gasförmiger Phase mit fest angeordneten Katalysatoren, auch als Festbett-Katalysatoren bezeichnet, durchgeführt; die kontinuierliche Arbeitsweise wird bevorzugt.

Bei diskontinuierlicher Verfahrensführung verwendet man, bezogen auf TCD-Dialdehyd, 1 bis 10, vorzugsweise 2 bis 6 Gew.-% Nickel in Form der vorstehend beschriebenen Katalysatoren. Bei kontinuierlicher Arbeitsweise setzt man je Liter Katalysator und Stunde etwa 0,05 bis etwa 5,0 kg des TCD-Dialdehyds ein, bevorzugt werden etwa 0,1 bis 2,0 kg TCD-Dialdehyd je Liter Katalysator und Stunde.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. In jedem Fall ist dafür Sorge zu tragen, dass das Hydriergas frei von Katalysatorgiften wie Schwefelverbindungen oder Kohlenmonoxid in schädlichen Mengen ist.

Das Rhodium aus dem nicht abgetrennten Hydroformylierungskatalysator schlägt sich nahezu vollständig auf dem Hydrierkatalysator nieder. Es kann nach bekannten Verfahren zurückgewonnen werden.

Es empfiehlt sich, in der Hydrierstufe Lösungs- oder Verdünnungsmittel einzusetzen, die reine Substanzen aber auch Substanzgemische sein können. Beispiele für geeignete Lösungs- oder Verdünnungsmittel sind lineare oder cyclische Ether wie Tetrahydrofuran oder Dioxan, sowie aliphatische Alkohole wie Methanol, 2-Ethylhexanol oder i-Butanol. Die Menge des eingesetzten Lösungs- oder Verdünnungsmittels kann entsprechend den apparativen und verfahrenstechnischen Gegebenheiten frei gewählt werden, im allgemeinen setzt man Lösungen ein, die 10 bis 75 Gew.-% TCD-Dialdehyd enthalten. Besonders bewährt hat es sich im Rahmen des erfindungsgemäßen Verfahrens, das aus der Hydroformylierungsstufe anwesende Verdünnungsmittel als Lösungs- oder Verdünnungsmittel zu verwenden. In diesem Fall ist, bezogen auf das Gewicht des TCD-Dialdehyds, zweckmäßig die 1-bis 10-fache, vorzugsweise die 1- bis 5-fache Menge des Lösungs- und Verdünnungsmittel anwesend oder wird zugesetzt.

Die Gewinnung des reinen TCD-Alkohol DM erfolgt nach konventionellen Destillationsverfahren. Restmengen des in der Hydroformylierungsstufe eingesetzten Rhodiums fallen im Destillationsrückstand an und werden nach bekannten Verfahren zurückgewonnen.

Im Folgenden wird das erfindungsgemäße Verfahren anhand einiger Beispiele näher erläutert.

### Vergleichsversuch

Die Hydroformylierung von Dicyclopentadien erfolgte nach dem aus GB-1,170,226 bekannten Verfahren unter Rhodiumkatalyse in Gegenwart von Toluol. Die Umsetzung einer 65 Gew.-%igen toluolischen Lösung an Dicyclopentadien erfolgte bei einer Temperatur von 130°C und einem Druck von 26 MPa in Gegenwart von 20 ppm Rhodium.

Anschließend wurde das Rohprodukt an einem Nickelfestbett-Katalysator bei einer Temperatur von 120°C und einem Druck von 10,0 MPa hydriert und anschließend destilliert.

Die Ausbeute an TCD-Alkohol DM betrug 70,5 %, bezogen auf eingesetztes Dicyclopentadien.

### Beispiel 1

Es wurde analog Beispiel 1 gearbeitet mit der einzigen Ausnahme, dass dem Einsatzgemisch zur Dicyclopentadienhydroformylierung 1,60 Gew.-% Wasser, bezogen auf das Einsatzgemisch, zugesetzt wurden.

Das nach der Hydroformylierung erhaltene Rohprodukt wurde ohne weitere Reinigung analog dem Vergleichsversuch hydriert und anschließend destilliert.

Die Ausbeute an TCD-Alkohol DM betrug 80,3 %, bezogen auf eingesetztes Dicyclopentadien.

Überraschenderweise können wasserhaltige Hydroformylierungsprodukte der Dicyclopentadienhydroformylierung oder Hydroformylierungsgemische nach Wasserzusatz in der nachfolgenden Hydrierstufe mit ausgezeichneten Ausbeuten in TCD-Alkohol DM überführt werden.

## Patentansprüche

1. Verfahren zur Herstellung von 3(4),8(9)-Dihydroxymethyltricyclo[5.2.1.0^{2.6}]decan durch Hydrierung der Hydroformylierungsprodukte des Dicyclopentadiens, **dadurch gekennzeichnet, dass** man die Hydroformylierungsprodukte in homogener organischer Phase in Gegenwart von homogen gelösten Rhodiumkatalysatoren herstellt und ohne weitere Reinigung, ohne Katalysatorabtrennung und ohne Lösungsmittelabtrennung die Hydrierung in Gegenwart von Nickelkatalysatoren nach Zusatz von Wasser durchführt.

2. Verfahren nach Anspruch 1, **dadurch gekennzeichnet, dass** der Wasserzusatz vor oder während der Hydrierung erfolgt.

3. Verfahren nach Anspruch 1 oder 2, **dadurch gekennzeichnet, dass** man bei der Hydrierung Wasser dem Reaktionsgemisch auf einen Gehalt von 0,5 bis 5,0 Gew.-%, bezogen auf die gesamte Einsatzmenge, zusetzt.

4. Verfahren gemäß Anspruch 3, **dadurch gekennzeichnet, dass** man Wasser auf einen Gehalt von 1,0 bis 4,0 Gew.-%, bezogen auf die gesamte Einsatzmenge, zusetzt.

5. Verfahren nach einem oder mehreren der Ansprüche 1 bis 4, **dadurch gekennzeichnet, dass** die Hydrierung in Gegenwart aliphatischer Alkohole erfolgt.

6. Verfahren nach einem oder mehreren der Ansprüche 1 bis 5, **dadurch gekennzeichnet, dass** man die Hydrierung bei einer Temperatur von 70 bis 170°C und bei einem Druck von 1 bis 30 MPa durchführt.

7. Verfahren nach Anspruch 1, **dadurch gekennzeichnet, dass** die Hydroformylierung in Gegenwart von Wasser erfolgt.

8. Verfahren nach Anspruch 7, **dadurch gekennzeichnet, dass** Wasser in einer Menge von wenigstens 0,1 Gew.-%, bezogen auf den Gesamteinsatz, zugegen ist.

9. Verfahren nach Anspruch 8, **dadurch gekennzeichnet, dass** Wasser in einer Menge von wenigstens 0,5 Gew.-%, bezogen auf den Gesamteinsatz, zugegen ist.

10. Verfahren nach Anspruch 9, **dadurch gekennzeichnet, dass** der Wassergehalt 0,5 bis 2,5 Gew.-%, bezogen auf den Gesamteinsatz, beträgt.

11. Verfahren nach Anspruch 10, **dadurch gekennzeichnet, dass** der Wassergehalt 1,0 bis 2,0 Gew.-%, bezogen auf den Gesamteinsatz, beträgt.

## Claims

1. A process for preparing 3(4),8(9)-dihydroxymethyltricyclo[5.2.1.0^{2,6}]-decane by hydrogenation the hydroformylation products of dicyclopentadiene, wherein the hydroformylation products are obtained in homogeneous organic phase in the presence of homogeneously dissolved rhodium catalysts and without further purification, without catalyst removal and without solvent removal the hydrogenation is carried out in the presence of nickel catalysts after addition of water.

2. The process as claimed in claim 1, wherein the water is added before or during the hydrogenation.

3. The process as claimed in claim 1 or 2, wherein water is added to the reaction mixture in the hydrogenation to a content of from 0.5 to 5.0% by weight, based on the total use amount.

4. The process as claimed in claim 3, wherein water is added to a content of from 1.0 to 4.0% by weight, based on the total use amount.

5. The process as claimed in one or more of claims 1 to 4, wherein the hydrogenation is effected in the presence of aliphatic alcohols.

6. The process as claimed in one or more of claims 1 to 5, wherein the hydrogenation is carried out at a temperature of from 70 to 170°C and at a pressure of from 1 to 30 MPa.

7. The process as claimed in claim 1, wherein the hydroformylation is effected in the presence of water.

8. The process as claimed in claim 7, wherein water is present in an amount of at least 0.1 % by weight, based on the total use.

9. The process as claimed in claim 8, wherein water is present in an amount of at least 0.5% by weight, based on the total use.

10. The process as claimed in claim 9, wherein the water content is from 0.5 to 2.5% by weight, based on the total use.

11. The process as claimed in claim 10, wherein the water content is from 1.0 to 2.0% by weight, based on the total use.

## Revendications

1. Procédé pour la préparation de 3(4),8(9)-dihydroxyméthyltricyclo[5.2.1.0^{2.6}]décane par hydrogénation des produits d'hydroformylation du dicyclopentadiène, **caractérisé en ce qu'**on prépare les produits d'hydroformylation en phase organique homogène en présence de catalyseurs au rhodium dissous de façon homogène et, sans autre purification, sans séparation de catalyseur et sans séparation de solvant on effectue l'hydrogénation en présence de catalyseurs au nickel après addition d'eau.

2. Procédé selon la revendication 1, **caractérisé en ce que** l'addition d'eau s'effectue avant ou pendant l'hydrogénation.

3. Procédé selon la revendication 1 ou 2, **caractérisé en ce que** lors de l'hydrogénation on ajoute de l'eau au mélange réactionnel jusqu'à une teneur de 0,5 à 5,0 % en poids, par rapport à la quantité totale de la charge initiale.

4. Procédé selon la revendication 3, **caractérisé en ce qu'**on ajoute de l'eau jusqu'à une teneur de 1,0 à 4,0 % en poids, par rapport à la quantité totale de la charge initiale.

5. Procédé selon une ou plusieurs des revendications 1 à 4, **caractérisé en ce que** l'hydrogénation s'effectue en présence d'alcools aliphatiques.

6. Procédé selon une ou plusieurs des revendications 1 à 5, **caractérisé en ce qu'**on effectue l'hydrogénation à une température de 70 à 170 °C et sous une pression de 1 à 30 MPa.

7. Procédé selon la revendication 1, **caractérisé en ce que** l'hydroformylation s'effectue en présence d'eau.

8. Procédé selon la revendication 7, **caractérisé en ce que** l'eau est ajoutée en une quantité d'au moins 0,1 % en poids, par rapport à la charge initiale totale.

9. Procédé selon la revendication 8, **caractérisé en ce que** l'eau est ajoutée en une quantité d'au moins 0,5 % en poids, par rapport à la charge initiale totale.

10. Procédé selon la revendication 9, **caractérisé en ce que** la teneur en eau vaut de 0,5 à 2,5 % en poids, par rapport à la charge initiale totale.

11. Procédé selon la revendication 10, **caractérisé en ce que** la teneur en eau vaut de 1,0 à 2,0 % en poids, par rapport à la charge initiale totale.
